(19) Europäisches Patentamt
European Patent Office
Office européen des brevets

(11) EP 1 339 818 B1

# (12) EUROPÄISCHE PATENTSCHRIFT

(45) Veröffentlichungstag und Bekanntmachung des Hinweises auf die Patenterteilung:
**11.04.2007 Patentblatt 2007/15**

(21) Anmeldenummer: **01991809.3**

(22) Anmeldetag: **07.12.2001**

(51) Int Cl.:
***C11D 3/28*** (2006.01) ***C11D 3/22*** (2006.01)
***A61K 8/64*** (2006.01) ***A61K 8/65*** (2006.01)
***A61Q 5/02*** (2006.01) ***A61Q 19/10*** (2006.01)
***A23J 3/32*** (2006.01) ***A01N 25/30*** (2006.01)

(86) Internationale Anmeldenummer:
**PCT/EP2001/014423**

(87) Internationale Veröffentlichungsnummer:
**WO 2002/046342 (13.06.2002 Gazette 2002/24)**

(54) **REINIGUNGSWIRKSAME, GRENZFLÄCHENAKTIVE KOMBINATION AUS NACHWACHSENDEN ROHSTOFFEN MIT HOHER FETTLÖSEKRAFT**

INTERFACE-ACTIVE COMBINATION, WHICH IS EFFECTIVE IN CLEANING, WHICH IS COMPRISED OF RENEWABLE RAW MATERIALS, AND WHICH HAS A HIGH GREASE SOLUBILIZING POWER

COMBINAISON NETTOYANTE TENSIOACTIVE CONSTITUEE DE MATIERES PREMIERES RENOUVELABLES A FORT POUVOIR NETTOYANT

(84) Benannte Vertragsstaaten:
**AT BE CH CY DE DK ES FI FR GB GR IE IT LI LU MC NL PT SE TR**

(30) Priorität: **08.12.2000 DE 10061280**

(43) Veröffentlichungstag der Anmeldung:
**03.09.2003 Patentblatt 2003/36**

(73) Patentinhaber: **Novaprot GmbH**
**92551 Stulln (DE)**

(72) Erfinder: **METZGER, Wolfgang**
**99425 Weimar (DE)**

(74) Vertreter: **Dey, Michael et al**
**Weickmann & Weickmann**
**Patentanwälte**
**Postfach 86 08 20**
**81635 München (DE)**

(56) Entgegenhaltungen:
**WO-A-01/17488**
**WO-A-01/60916**
**WO-A-99/57154**
**US-A- 3 594 324**
**US-A- 3 891 571**
**US-A- 4 826 535**
**US-A- 5 225 095**
**US-A- 5 389 676**

EP 1 339 818 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft eine grenzflächenaktive Zusammensetzung aus nachwachsenden Rohstoffen.

Wasch- und Reinigungsmittel sowie industriell eingesetzte Netzmittel oder Tenside sollten möglichst aus umweltverträglichen Stoffen hergestellt sein, um die Umwelt und hier insbesondere die aquatischen Systeme nicht oder nur minimal zu belasten. Weiterhin sollten sie wünschenswerterweise aus natürlichen, nachwachsenden Rohstoffen und nicht aus Erdöl auf petrochemischem Wege hergestellt sein.

Es ist bekannt, dass ein geringer Zusatz von kationischen Tensiden zu Tensidkombinationen aus anionischen und nicht-ionischen Tensiden eine bessere Reinigungs- und Waschwirkung sowie eine geringe Beschichtung von Oberflächen (Glanzwirkung) bewirkt. Bei Wäsche wird durch Zusatz von kationischen Tensiden zudem ein besserer Griff erzeugt. Üblicherweise werden auf 3 bis 4 Teile anionisches Tensid etwa 1 Teil nicht-ionisches Tensid eingesetzt, wobei die Menge an kationischen Tensiden bis zu ca. 10 % der gesamten Tensidmenge beträgt. Durch Zusätze von Salzen mit verschiedenen Ladungen können die Eigenschaften von Tensidkombinationen weiter beeinflusst werden. Durch die Verwendung von Komplexbildnern oder Schmutzträgergelen (z.B. Carboxymethylcellulose (CMC)) kann weiterhin die Vergrauung von Oberflächen oder Wäsche minimiert werden.

Kationenaktive Tenside ziehen im sauren Bereich auf Wäsche bzw. Gegenstände auf und werden deshalb als Antistatika, Weichspüler, Hydrophobiermittel oder Appreturen eingesetzt.

Weiterhin ist bekannt, dass einige Proteine oder Proteinhydrolysate in wässriger Lösung ampholytisch wirken können. Sie können modellhaft mit der Formel

$$S^- - R - B^+$$

dargestellt werden, wobei R das Grundgerüst, insbesondere einen hydrophoben Rest und $S^-$ und $B^+$ die unterschiedlichen Ladungen darstellen. Solche Proteine oder Proteinhydrolysate besitzen somit den für seifenartige Systeme typischen Aufbau aus hydrophoben und hydrophilen Strukturelementen, mit dem Unterschied, dass es sich nicht um kleine Moleküle mit niedrigem Molekulargewicht, sondern um hochmolekulare bzw. polymere Moleküle handelt. Polymere ampholytische Proteinstrukturen, die hydrophile und hydrophobe Bereiche im Molekül enthalten, sind in der Lage, kolloidale Systeme, wie Dispersionen oder Emulsionen, in erster Linie sterisch zu stabilisieren.

Eine sterische Stabilisierung tritt auf, wenn kolloidale Teilchen von einer Hülle aus Makromolekülen umgeben sind und sich in einem guten Lösungsmittel befinden. Dabei dient eine Komponente bzw. ein Molekülsegment des Polymers als Ankergruppe und ist weitgehend unlöslich im Dispersionsmittel, z.B. in Wasser. Im Fall von Proteinen sind dies die hydrophoben Bereiche des Moleküls. Eine weitere Komponente bzw. ein weiteres Molekülsegment des Polymers dient als stabilisierender Teil des Makromoleküls und ist in dem Lösungs- oder Dispersionsmittel, z.B. in Wasser, löslich. Im Fall von Proteinen und Wasser als Dispersionsmittel sind dies die polaren Bereiche des Proteinmoleküls.

US 3,594,324 offenbart Waschmittelzusammensetzungen, welche ein Detergenz, einen Detergenzbuilder und einen Vergrauungsinhibitor umfassen. Als Detergenz wird bevorzugt ein lineares Alkylbenzolsulfonat eingesetzt, als Vergrauungsinhibitor dient eine Mischung aus Carboxymethylcellulose und einem Gelatineprotein.

US 5,225,095 beschreibt die Herstellung eines stabilen Schaumkonzentrats. Als Einzelkomponenten werden ein Proteinhydrolysat, mehrwertige Kationen sowie ein wasserlösliches Polymer auf Zuckerbasis verwendet.

US 4,826,535 offenbart eine Anstrichfarbenzusammensetzung, welche sich durch Waschen leicht aus Kleidungsstücken entfernen lässt. Neben Pigmenten, Weichmachern, Pigmentextendem und Konservierungsmitteln enthält die Zusammensetzung optional ein flüssiges Protein oder einen als Verdickungsmittel eingesetzten polymeren Naturstoff.

US 3,891,571 beschreibt schaumbildende Zusammensetzungen, welche insbesondere für Anwendungen im Pflanzenschutz geeignet sind. Zusammensetzungen dieser Art enthalten neben Molkeprodukten und Tensiden wasserdispergierbare Polymere, die als Viskositätsbuilder dienen.

WO 99/57154 offenbart Zusammensetzungen, die im Bereich der Textilpflege eingesetzt werden. Als Weichmacher wird ein modifiziertes Protein verwendet, welches eine Bindungssequenz für Cellulose aufweist.

In US 5,389,676 werden infektionshemmende Wasser-in-Öl- und Öl-in-Wasser-Emulsionen beschrieben, welche im kosmetischen Bereich Anwendung finden. Derartige Zusammensetzungen umfassen neben Tensiden weiterhin hydrophobe Verbindungen sowie Gelatine und Cellulosederivate, die als Emulsionshilfsstoffe zur Erhöhung der Viskosität dienen.

Für die Anwendung als grenzflächenaktive Substanzen in wässrigen Systemen besitzen polymere Proteinstrukturen allerdings einige Nachteile.

Zwar können die ionischen bzw. geladenen Gruppen in einem bestimmten Bereich des Proteinmoleküls angehäuft sein, im Vergleich zu monomeren grenzflächenaktiven Verbindungen, z.B. handelsüblichen Tensiden, sind sie

jedoch über einen größeren Bereich des Moleküls verteilt. Somit weisen ampholytische Proteinmoleküle eine geringere punktuelle Ladungsdichte als herkömmliche niedermolekulare Tenside auf und der stabilisierende Teil (der wasserlöslichkeitsvermittelnde Teil) des polymeren Eiweißmoleküls besitzt dadurch eine geringere Löslichkeit im Dispersionsmittel Wasser.

Darüber hinaus ist es erforderlich, dass die Proteine in wenigstens teilweise linearer Struktur vorliegen, um eine grenzflächenaktive Wirkung zu zeigen. Globuläre Proteine müssen deshalb erst an der Grenzfläche aufgefaltet werden, bevor sie stabilisierend wirken können. Somit können als grenzflächenaktive Verbindungen bisher nur von Natur aus überwiegend lineare Eiweißmoleküle eingesetzt werden, welche nur einen sehr geringen Anteil an Sekundärstrukturen aufweisen, welche nicht-lineare Bereiche bilden, wie etwa $\alpha$-Helix oder $\beta$-Faltblatt. Lineare Proteine haben jedoch den Nachteil, dass es bei Anwesenheit mehrwertiger Ionen, z.B. $Ca^{2+}$, zu intermolekularen elektrostatischen Wechselwirkungen bzw. Brückenbildungen zwischen polyanionischen Eiweißmolekülen kommen kann, die zu einer Abnahme der Löslichkeit in Wasser und im äußersten Fall zu einer Flockung des Proteins führen.

Ein weiterer Nachteil verschiedener Proteine für den Einsatz als grenzflächenaktive Verbindung bzw. als Emulgator oder Dispergierhilfsmittel in wässrigen Systemen ist deren oftmals pH-Wertabhängiges Lösungsverhalten. Üblicherweise nimmt die Löslichkeit von ampholytischen Proteinen bereits im schwach sauren Bereich, der für viele Reinigungsanwendungen wünschenswert ist; deutlich ab.

Eine Aufgabe der vorliegenden Erfindung war es deshalb, eine reinigungswirksame, grenzflächenaktive Kombination aus nachwachsenden Rohstoffen mit hoher Fettlösekraft bereitzustellen.

Diese Aufgabe wird erfindungsgemäß gelöst durch eine grenzflächenaktive Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen, und
d) mindestens einer Aminosäure.

Ein weiterer Gegenstand der Erfindung ist die Verwendung einer Kombination aus (a), (b), (c) und (d) als grenzflächenaktive Zusammensetzung.

Der Komplexbildner ist insbesondere in der Lage, mehrwertige, z.B. 2-, 3-, 4-, 5- oder/und 6-wertige Ionen, bevorzugt Kationen, wie z.B. Metallionen zu komplexieren, zu binden oder/und auszufällen.

Das Gewichtsverhältnis der Komponenten a) : b) beträgt bevorzugt 3:1 bis 1:3, insbesondere 2:1 bis 1:3 und besonders bevorzugt 1,5:1 bis 1:2.

Die erfindungsgemäße Zusammensetzung, die aus nachwachsenden Rohstoffen gebildet ist, weist aufgrund ihres sehr guten emulgierenden, dispergierenden sowie grenzflächenaktiven und filmbildenden Charakters selbst in geringsten Dosierungen hervorragende tensidische Eigenschaften auf.

Darüber hinaus weisen die erfindungsgemäßen Zusammensetzungen eine hervorragende Benetzungswirkung auf. Durch zusätzliche Verwendung von Aminosäuren können die beschriebenen dispergierenden und filmbildenden Eigenschaften der Zusammensetzung weiterhin verstärkt werden. Geeignete Aminosäuren sind insbesondere die 20 natürlich vorkommenden Aminosäuren. Besonders bevorzugt wird eine Aminosäure mit negativ geladenen Seitenketten, wie z.B. Glutaminsäure oder Asparaginsäure. Die Anlagerung der Aminogruppe (positiv geladen) an die negativ geladenen Gruppen (z.B. Aminosäurereste, Carbonsäuregruppen) des Proteins (Komponente a)) führt dabei zu einer weiteren Erhöhung der Ladungsdichte des Proteins.

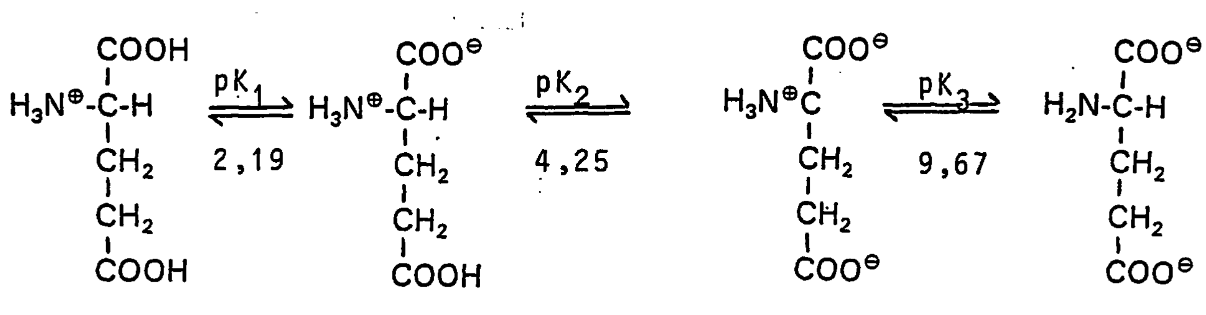


Im Bereich pH 4,25 bis pH 9,67, insbesondere bei pH 6 bis 8 oder bei pH 6 bis 7, führt dies zu einer Steigerung der Anionaktivität:

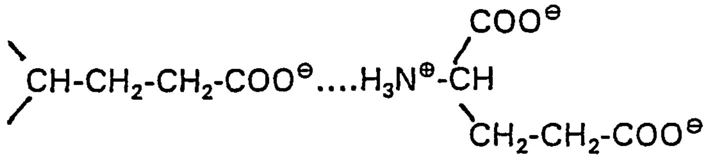

Eine Steigerung der Anionaktivität kann auch durch Zugabe von Citrat (mit drei Säuregruppen) erhalten werden. Die Anlagerung erfolgt dabei an positiv geladene Gruppen des Proteins, z.B. an Lysinreste.

Vor allem die sehr hohe Fettlösekraft der erfindungsgemäßen Zusammensetzung erlaubt eine sehr geringe Wirkstoffdosierung für verschiedene Reinigungszwecke, was weiterhin zu einer Reduzierung des Eintrags von Waschmittelinhaltsstoffen in die Umwelt, insbesondere in die aquatische Umwelt, führt. Die Inhaltsstoffe der erfindungsgemäßen Zusammensetzung sowie deren Kombinationen sind zudem leicht biologisch abbaubar. Die biologische Abbaubarkeit kann beispielsweise gemäß dem $CO_2$-Entwicklungstest nach 67/548/EWG bestimmt werden, einem Test zur Untersuchung der vollständigen aeroben biologischen Abbaubarkeit von Chemikalien in wässrigen Medien. Unter diesen Testbedingungen wurde für die erfindungsgemäße Zusammensetzung innerhalb von 21 Tagen eine Abbaubarkeit von 100 % erreicht.

Weiterhin ist die erfindungsgemäße Zusammensetzung sehr hautfreundlich, wie sich in dermatologischen Untersuchungen gezeigt hat. Sowohl der Patch-Test am Menschen als auch die Prüfung auf Hautreizung beim Menschen im modifizierten Duhring-Kammer-Test, ließen kein Irritationspotenzial erkennen. Auch bei einem Test auf membranirritierende Eigenschaften zeigten die erfindungsgemäßen Zusammensetzungen keine Reizung.

Durch die Zugabe von polaren, anionisch oder nicht-ionisch wirkenden Naturstoffen, insbesondere durch geeignete Polysaccharide sowie von Komplexbildnern erhalten die in der erfindungsgemäßen Zusammensetzung enthaltenen ampholytischen Proteine bzw. Peptide oder Proteinhydrolysate grenzflächenaktive Eigenschaften, die eine Anwendung in Wasch- und Reinigungsmitteln erst ermöglichen. In der erfindungsgemäßen Zusammensetzung werden die Eigenschaften von ampholytischen Proteinen durch Zugabe von polaren, anionisch oder nicht-ionisch wirkenden Naturstoffen, insbesondere durch geeignete Polysaccharide, verbessert. In der erfindungsgemäßen Zusammensetzung ist insbesondere die Reinigungs, Emulions-, Dispersions- und Schmutzträgerwirkung sowie das Aufziehvermögen von ampholytischen Proteinen verbessert. In der erfindungsgemäßen Zusammensetzung zeigt die eingesetzte Kombination aus ampholytischem Protein, polarem anionischen oder/und nicht-ionischem polymeren Naturstoff sowie Komplexbildner eine Benetzungswirkung, Grenzflächenaktivität und Emulgierfunktion und somit eine Reinigungswirksamkeit.

Ohne dass beabsichtigt ist, sich auf einen Wirkmechanismus festzulegen, wird vermutet, dass es über elektrostatische Wechselwirkung zu einer Anlagerung der polaren, anionisch oder nicht-ionisch wirkenden Verbindungen an die polaren Molekülbereiche der Proteine kommt. Dies hat eine erhöhte Ladungsdichte der polaren Bereiche der Protein- bzw. Peptidmoleküle zur Folge, die zu einer verbesserten Löslichkeit dieser Bereiche in wässrigen Dispersionsmitteln führt. Dadurch kommt es zu einer deutlichen Erhöhung der Grenzflächenaktivität dieser Aggregate gegenüber dem freien, nativen Protein. Außerdem ist durch die deutlich verbesserte Löslichkeit der polaren Bereiche der Protein- bzw. Peptidmoleküle sowie durch das größere Volumen der Aggregate (bestehend aus ampholytischem Protein bzw. Peptid und polymerem Naturstoff) die Fähigkeit zur sterischen Stabilisierung weiter erhöht. Somit weisen die erfindungsgemäßen grenzflächenaktiven Zusammensetzungen gegenüber bekannten monomeren Tensidmolekülen und gegenüber freien ampholytischen Proteinen ein erhöhtes sterisches Stabilisierungsvermögen auf. Während die bisher bekannten Tenside überwiegend nur elektrostatisch stabilisierte Systeme bilden konnten, sind die erfindungsgemäßen Kombinationen aufgrund ihres polymeren Charakters auch zur sterischen Stabilisierung kolloidaler Systeme geeignet.

Dies bringt den Vorteil mit sich, dass sterisch stabilisierte Systeme weitgehend salzunabhängig sind und somit die kritischen Salzkonzentrationen für die erfindungsgemäßen grenzflächenaktiven Zusammensetzungen für viele Anwendungen, wie etwa im Pflanzenschutz, weit oberhalb derjenigen elektrostatisch stabilisierter Systeme liegen. So können die erfindungsgemäßen grenzflächenaktiven Zusammensetzungen Salzkonzentrationen von bis zu 0,2 mol/l, beispielsweise von bis zu 0,2 mol/l $Ca^{2+}$ enthalten. Für die Anwendung in Waschmitteln oder Reinigungszusammensetzungen werden bevorzugt geringe Salzkonzentrationen eingestellt und gegebenenfalls Ionen durch Komplexbildner eingefangen.

Ein weiterer Vorteil der erfindungsgemäßen grenzflächenaktiven Zusammensetzung besteht darin, dass die aus ampholytischem Protein bzw. Peptid und polymerem Naturstoff unter Anwesenheit von Komplexbildner gebildeten Addukte oder Aggregate auch aufgrund ihrer räumlichen Struktur deutlich weniger anfällig gegenüber Ausflockung von Proteinen in Gegenwart mehrwertiger Ionen sind. Vielmehr werden in der erfindungsgemäßen Kombination die komplexbildenden Eigenschaften der Proteine verstärkt, sodass die bei Reinigungsvorgängen oftmals störende Wasserhärte von den gebildeten Aggregaten aus ampholytischem Protein oder polymerem Naturstoff aufgenommen bzw. verringert

wird.

Komplexbildner, z.B. Salze, die zur Komplexbildung neigen, wie z.B. Polyphosphate, Gluconate oder Citrate, erhöhen auf der anderen Seite die Reinigungswirkung der beschriebenen grenzflächenaktiven Zusammensetzung, da sie Ca- oder Mg-Ionen aus dem Protein lösen und dadurch die Löslichkeit des Proteins erhöhen. In einer Kombination aus ampholytischem Protein bzw. Peptidgemisch und polymerem Naturstoff in Anwesenheit eines Komplexbildners wird die Reinigungswirksamkeit durch die Anwesenheit von Komplexbildner deutlich erhöht, da sie mehrwertige Ionen, wie z.B. $Ca^{2+}$ oder $Mg^{2+}$, die ansonsten zu einer Vernetzung der Proteinmoleküle über elektrostatische Wechselwirkungen führen würden, aus dem Protein lösen und dadurch die Löslichkeit des Proteins, aber auch die Grenzflächenaktivität der Addukte aus ampholytischem Protein bzw.

Peptid und polymerem Naturstoff deutlich erhöhen. Ein Zusatz von Aminosäuren, insbesondere von Aminosäuren mit negativ geladenen Seitengruppen führt zu einer weiteren Verbesserung dieser Eigenschaften.

Weiterhin ist es möglich, durch die erfindungsgemäße Verwendung eines polaren anionischen polymeren Naturstoffs, welcher negative Ladungen enthält, oder/und eines anionischen Tensids den isoelektrischen Punkt des Gesamtsystems so zu verschieben, dass es im sauren Bereich nicht zu einer Ausflockung der Proteine kommt. Dadurch kann das bei verschiedenen Proteinen nachteilige pH-Wert-abhängige Lösungsverhalten deutlich verbessert werden, was zur Folge hat, dass die erfindungsgemäße Zusammensetzung auch in sauren Anwendungen erfolgreich eingesetzt werden kann, wie etwa in Bad- oder WC-Reinigern. Somit kann die erfindungsgemäße Zusammensetzung auch in sauren Reinigern, z.B. in Kombination mit Zitronen- oder Essigsäure, verwendet werden.

Die beste Reinigungswirkung der erfindungsgemäßen Zusammensetzungen wurde bei pH-Werten zwischen 6,0 und 7,0 beobachtet.

Es wurde festgestellt, dass sich in den erfindungsgemäßen Zusammensetzungen die Eigenschaften der Ausgangsrohstoffe synergistisch verstärken und dass die Zusammensetzungen insbesondere eine hohe Grenzflächenaktivität bzw. Oberflächenaktivität aufweisen. Eine weitere positive Eigenschaft der erfindungsgemäßen Zusammensetzung ist deren hohe Substantivität. Dies ermöglicht den Einsatz der erfindungsgemäßen Zusammensetzung auch bei der Textilbeschichtung, welche zu einer Ausstattung von Textilien mit hydrophilem Charakter führt.

Weiterhin wurde festgestellt, dass mit den erfindungsgemäßen Zusammensetzungen bereits eine Weichspülerwirkung erzielt wird, ohne dass der Zusatz von Weichmachern, wie z.B. Fettsäureamidpolyglykolether erforderlich ist.

Bevorzugt wird ein ampholytisches Protein oder Peptid eingesetzt, welches sowohl hydrophile als auch hydrophobe Bereiche enthält und besonders bevorzugt kann ein aus ampholytischen Proteinen durch Hydrolyse hergestelltes Peptidgemisch eingesetzt werden. Solche Moleküle besitzen den für seifenartige Systeme typischen Aufbau aus hydrophoben und hydrophilen Strukturelementen, also Strukturelementen, die in unpolaren bzw. polaren Lösungsmitteln löslich sind. Ampholytische Proteine, die sowohl hydrophile als auch hydrophobe Bereiche enthalten, können auch als amphophile Proteine bezeichnet werden. Das Verhältnis von hydrophoben zu hydrophilen Bereichen kann bevorzugt von 10 : 90 bis 90 : 10, mehr bevorzugt von 30 : 70 bis 70 : 30 betragen.

Als Ampholyt werden allgemein Verbindungen bezeichnet, die sowohl saure als auch basische hydrophile Gruppen besitzen und sich somit je nach Bedingung sauer oder basisch verhalten. Das erfindungsgemäß eingesetzte ampholytische Protein oder Peptid enthält mindestens 1, bevorzugt mindestens 5, mehr bevorzugt mindestens 10 basisch wirkende Gruppierungen und mindestens 1, bevorzugt mindestens 5, mehr bevorzugt mindestens 10 sauer wirkende Gruppierungen. Geeignete basisch wirkende Gruppierungen sind beispielsweise Aminogruppen, während geeignete sauer wirkende Gruppierungen beispielsweise Carbonsäuregruppen sind.

Das Verhältnis von sauren zu basischen Gruppierungen kann über einen großen Bereich variieren und beträgt bevorzugt 20 : 1 bis 1 : 20, mehr bevorzugt von 10 : 1 bis 1 : 10 und am meisten bevorzugt 5 : 1 bis 1 : 5.

Beispiele erfindungsgemäß einsetzbarer ampholytischer Proteine umfassen tierische Proteine, wie z.B. Milchproteine (Milcheiweiß) oder pflanzliche Proteine, wie etwa Sojaproteine, Lupinenproteine, Weizenproteine oder/und Erbsenproteine oder bakterielle Proteine. Bevorzugt wird das Milcheiweiß in Form von Casein oder eines Caseinats, beispielsweise als Natriumcaseinat, verwendet. Es ist aber auch möglich, einen Eiweißträger mit geringerer Proteinkonzentration, wie z.B. Magermilchpulver, einzusetzen.

Der Begriff "ampholytisches Protein", wie hierin verwendet, umfasst auch Proteinhydrolysate. Als Ausgang für die durch Hydrolyse hergestellten Peptidgemische werden Proteine natürlicher Herkunft verwendet, die bevorzugt wasserlöslich sein sollten, sowohl pflanzlichen, tierischen aber auch bakteriellen Ursprungs sein können und keine proteolytische Enzymaktivität mehr besitzen. Beispiele erfindungsgemäß zur Herstellung der Peptidgemische einsetzbarer ampholytischer Proteine umfassen Milchproteine, Collagene, Gelatine, Albumine, Eiproteine, Sojaproteine, Lupinenproteine, Erbsenproteine, Weizenproteine, Kartoffelproteine, Rapsproteine. Bevorzugt wird hydrolysiertes Milcheiweiß, z.B. in Form von Caseinhydrolisat verwendet. Es ist aber auch möglich, bei Anwendungen im sauren oder alkalischen Bereich, die aufgrund des vorliegenden pH-Werts zu einer Proteinhydrolyse führen, in der entsprechenden Formulierung ein natives Protein einzusetzen, welches dann in situ hydrolysiert wird. Weiterhin können auch Eiweißträger mit geringerer Proteinkonzentration, wie z.B. Magermilchpulver, verwendet werden. Erfindungsgemäß geeignete Proteine bzw. Peptide weisen insbesondere ein Molekulargewicht $\geq$ 500 Da, bevorzugt $\geq$ 1.000 Da, mehr bevorzugt $\geq$ 10.000 Da und sogar $\geq$

100.000 Da auf und können ein Molekulargewicht von einigen Mio Da, insbesondere bis zu 2 Mio Da aufweisen.

Als polarer polymerer Naturstoff wird bevorzugt ein Polysaccharid verwendet. Beispiele geeigneter anionischer Polysaccharide sind Glucane, Galactane, Polyuronsäuren usw., die eine entsprechende Anionenaktivität aufweisen. Beispiele für Glucane sind Xanthan, Cellulose und deren Derivate, wie Carboxymethylcellulose, Dextrane, Stärkederivate u.dgl. Als Galactane sind beispielhaft Carrageene, Agar u.dgl. aufzuführen. Beispiele für Polyuronsäuren sind Alginsäuren, Alginate, Pektine usw. Als nicht-ionische Polysaccharide können Galactomannane, wie z.B. Guarkernmehl oder Johannisbrotkernmehl aber auch Glucomannane, wie z.B. Amylopektin oder Konjac Gummi verwendet werden.

Die anionischen Polysaccharide ersetzen die in üblichen Reinigungsformulierungen enthaltenen anionaktiven Tenside und die nicht-ionischen Polysaccharide übernehmen aufgrund ihrer Struktur die Aufgaben der in diesen Formulierungen enthaltenen nicht-ionischen Tenside. Sie sind somit auch teilweise durch übliche anionaktive bzw. nichtionische Tenside ersetzbar, z.B. bei Anwendungen, in denen ein höheres Schaumbildungsvermögen erwünscht ist. Anwendungen, bei denen ein Schäumen erwünscht ist, sind z.B. Reinigungsmittel und kosmetische Reinigungsprodukte, wie z.B. Duschgel, Shampoo oder Handwaschmittel.

Anwendungen, bei denen ein Schäumen nicht erforderlich ist sind z.B. Maschinenwaschmittel, wie etwa Wollwaschmittel, Buntwaschmittel, Geschirrmaschinenreiniger, saure Reiniger, im Pflanzenschutz oder im Baubereich.

Die Menge an Tensid zu polymerem Naturstoff (Gew./Gew.) beträgt in den erfindungsgemäßen Zusammensetzungen bevorzugt ≤ 20:1, insbesondere ≤ 15:1, ≤ 10:1, ≤ 8:1 und besonders bevorzugt ≤ 5:1. Sie kann aber sogar ≤ 1:1, ≤ 0,5:1 1 oder ≤ 0,1:1 1 betragen und es ist möglich, Formulierungen zu bilden, die frei von herkömmlichen Tensiden sind. Für Anwendungen mit erwünschter Schaumbildung können Tenside bevorzugt in einer Menge von 5:1 bis 15:1, bezogen auf die polymeren Naturstoffe eingesetzt werden. Für Anwendungen ohne Schaumbildung beträgt das Verhältnis Tensid zu polymerem Naturstoff bevorzugt von 0,1:1, insbesondere von 1:1 bis 5:1, insbesondere bis 2: 1. Die angegebenen höheren Mengen an Tensid (z.B. 10:1 oder mehr), kommen insbesondere in der Kosmetik zur Anwendung während für andere Einsatzgebiete geringere Mengen an Tensid (z.B. 5:1 oder weniger) ausreichend sind.

Als Komplexbildner eignen sich wasserlösliche Salze, die in der Lage sind zwei- bzw. mehrwertige Ionen stärker zu binden als die verwendeten Proteine bzw. polymeren Naturstoffe. Als Beispiele können zwei- oder mehrbasische Carbonsäuren sowie deren Salze wie z.B. Citronensäure oder Gluconsäure sowie deren Alkalisalze, aber auch andere Komplexbildner, wie Natriumaluminiumsilikate, Alkalisilikate, Ethylendiamintetraessigsäure sowie deren Alkalisalze und Polyphosphate oder Phosphonate aufgeführt werden. Aus ökologischer Sicht sind die aufgeführten Carbonsäuren bzw. deren Alkalisalze bevorzugt einzusetzen. Die größte Wirksamkeit wurde beobachtet, wenn die verwendeten Komplexbildner mit den aus dem System zu entfernenden mehrwertigen Ionen in Wasser unlösliche Salze bilden. Als Beispiele können Komplexbildner, wie Oxalsäure sowie deren Alkalisalze oder ortho-Phosphorsäure, sowie deren Alkalisalze aufgeführt werden.

Verbindungen, die in der Lage sind, mehrwertige Ionen, insbesondere Kationen auszufällen, sind wasserlösliche Verbindungen, welche mit mehrwertigen Ionen, z.B. mit $Ca^{2+}$-Ionen unlösliche Salze bilden. Beispiele solcher Verbindungen sind $Na_2SO_4$ oder $Na_2CO_3$.

Die Komplexbildner bzw. Verbindungen, die in der Lage sind, mehrwertige Ionen auszufällen, haben insbesondere die Funktion, Ionen, wie z.B. $Ca^{2+}$-Ionen oder $Mg^{2+}$-Ionen zu binden. Diese Ionen können ansonsten Verbrükkungen zwischen Molekülketten der Proteine bzw. Peptide der Komponente a) bilden und dadurch zur Bildung von Agglomeraten führen. Durch Zugabe der Komplexbildner wird dieser Mechanismus unterdrückt und die Löslichkeit des Proteins bzw. Peptids erhöht.

Aufgrund der sehr hohen Fettlösekraft der erfindungsgemäßen Zusammensetzung genügt eine geringe Wirkstoffdosierung, um zu zufriedenstellenden Reinigungseigenschaften zu gelangen. Bei Reinigungs- und Waschmitteln sind beispielsweise Konzentrationen von 0,01, insbesondere von 0,025 bis 0,04 % der erfindungsgemäßen Zusammensetzung bei der Endwanwendung ausreichend. Eine Formulierung kann dabei z.B. 0,1 bis 1 Gew.-% der erfindungsgemäßen Zusammensetzung enthalten und vor der Endanwendung entsprechend mit Wasser verdünnt werden. Eine ähnlich geringe Menge genügt als Reinigungsverstärkung in Verbindung mit Tensiden und/oder Reinigungschemikalien. Es können aber auch Zusammensetzungen hergestellt und eingesetzt werden, die je nach beabsichtigter Anwendung davon abweichende Konzentrationen der erfindungsgemäßen Zusammensetzung enthalten, z.B. von 0,01 Gew.-% bis 10 Gew.-%.

Die erfindungsgemäße, sich synergistisch verstärkende Kombination aus natürlich nachwachsenden Rohstoffen kann für bestimmte Anwendungen weiterhin mit einem Tensid, einem Salz oder/und einer Aminosäure versetzt werden. Durch die Zugabe von Salzen oder/und Aminosäuren kann der isoelektrische Punkt des Systems verschoben werden, um auch eine Anwendung im sauren Bereich, beispielsweise bei pH-Werten von < 7, bevorzugt < 5,5, mehr bevorzugt < 4 zu erreichen. Außerdem kann durch Zugabe von Aminosäuren der substantive Charakter des Systems (Aufziehvermögen) weiter verbessert werden. Eine Verbesserung der Reinigungs- und Waschwirkung kann insbesondere durch einen geringen Zusatz von zwei- oder mehrbasischen Salzen, z.B. Silikaten, Sulfaten, Carbonaten, Carboxylaten, Gluconaten, Citraten oder anderen mehrbasischen Carbonsäuren erzielt werden.

Mit der erfindungsgemäßen grenzflächenaktiven Zusammensetzung kann man zusätzlich zur Reinigung eine

Beschichtung der gereinigten Materialien, wie etwa Textilien, Glas, Holz, Kunststoff usw., erzielen. Es wurde beobachtet, dass nach dem Reinigungs- bzw. Waschvorgang ein Polymerfilm zurückbleibt, der bei den gereinigten Flächen als Glanz und bei Wäsche als Appretur (Griffigkeit) in Erscheinung tritt.

Die Herstellung der erfindungsgemäßen Zusammensetzung kann nach dem Fachmann bekannten Methoden erfolgen. Bevorzugt werden das Protein, insbesondere wasserlösliches Milcheiweiß oder Peptidgemisch, insbesondere hydrolysiertes Milcheiweiß, und der polare polymere Naturstoff, insbesondere ein Polysaccharid, besonders bevorzugt Xanthan, sowie ein Komplexbildner, insbesondere ein Alkalicitrat und/oder ein Alkalioxalat miteinander trocken vermischt und dann in der gewünschten Dosierung in wässrige Systeme eingearbeitet. Bei der Herstellung von Wasch- und Reinigungsmitteln kann die erfindungsgemäße Zusammensetzung vor der Zugabe von weiteren Inhaltsstoffen in Wasser dispergiert und zur Quellung gebracht werden.

Das Verhältnis der Inhaltsstoffe a) und b) kann über einen weiten Bereich variieren. Der Anteil an ampholytischem Protein beträgt bevorzugt mindestens 10, mehr bevorzugt mindestens 25 und besonders bevorzugt mindestens 40 Gew.-% und bevorzugt höchstens 80, mehr bevorzugt höchstens 65 und am meisten bevorzugt höchstens 50 Gew.-%, während der Anteil an polarem polymeren Naturstoff bevorzugt mindestens 20, mehr bevorzugt mindestens 35, am meisten bevorzugt mindestens 50 Gew.-% und bevorzugt höchstens 90, mehr bevorzugt höchstens 75 und am meisten bevorzugt höchstens 60 Gew.-%, jeweils bezogen auf das Gesamtgewicht der Komponenten a) und b) ausmacht.

Durch Einstellen eines Gewichtsverhältnisses von a) zu b) von 3:1 bis 1:3 wird insbesondere die gewünschte Reinigungswirksamkeit erzielt.

Der Anteil an Komplexbildner in den Formulierungen hängt im Wesentlichen von der vorliegenden Wasserhärte und der durch die Anwendung zugefügten Wassermenge ab, sollte jedoch in der Regel mindestens dem Gehalt der in der Kombination aus den Substanzen a) und b) enthaltenen Menge an Protein bzw. Proteinhydrolysat entsprechen.

Wie oben bereits ausgeführt, kann die erfindungsgemäße Zusammensetzung neben der Kombination aus den Substanzen a) und b) und c) Komplexbilder, weitere Inhaltsstoffe und Additive umfassen, insbesondere Tenside, Salze, weitere Reininungschemikalien und insbesondere Lösungs- und Dispergiermittel, wie etwa Wasser oder wässrige Lösungen mit einem Gehalt an Wasser von mindestens 40 Gew.%, mehr bevorzugt mindestens 80 Gew.-%, sowie organische Lösungsmittel, wie etwa alkoholische Lösungen.

Ein weiterer Gegenstand der Erfindung ist ein Konzentrat, welches vor der Endanwendung mit Wasser oder einem anderen Lösungsmittel verdünnt wird, welches eine wie oben beschriebene grenzflächenaktive Zusammensetzung umfassend ein ampholytisches Protein und einen polaren polymeren Naturstoff in einer Konzentration von ≥ 1 Gew.-%, bevorzugt ≥ 10 Gew.-%, mehr bevorzugt ≥ 50 Gew.-% und besonders bevorzugt ≥ 90 Gew.-% enthält. Ein solches Konzentrat kann in fester oder flüssiger Form vorliegen.

Aufgrund der hohen Grenzflächenaktivität ist die erfindungsgemäße Zusammensetzung für zahlreiche Anwendungen geeignet.

Ein weiterer Gegenstand der Erfindung ist deshalb die Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen, und
d) mindestens einer Aminosäure,

als Reinigungsmittel, Waschmittel, Emulgiermittel oder/und Dispergiermittel.

Es wurde festgestellt, dass in gewissen Anwendungen die oben beschriebene stabilisierende Wirkung von Addukten aus ampholytischem Protein bzw. ampholytischen Peptidgemischen und polarem Molekül auch durch Verwendung von anionischen oder nicht-ionischen Tensiden in Kombination mit einem ampholytischen Protein erzielt werden können. Bevorzugt ist bei jeder der hierin genannten Anwendungen der Einsatz einer Zusammensetzung, welche einen polaren polymeren Naturstoff enthält. Es ist aber auch möglich, die anionischen Naturstoffe durch herkömmliche anionische Tenside bzw. die nicht-ionisch wirkenden Naturstoffe durch herkömmliche nicht-ionische Tenside teilweise zu ersetzen oder zu ergänzen, und immer noch vorteilhafte Wirkungen zu erzielen.

Die Kombination enthält mindestens einen Komplexbildner.

Eine solche grenzflächenaktive Zusammensetzung kann als Basis für verschiedene hochwirksame und umweltfreundliche Reinigerformulierungen, insbesondere als Haushaltsreiniger, eingesetzt werden. Ein wesentlicher Vorteil gegenüber bekannten Produkten ist die sehr hohe Fettlösekraft und die damit verbundene Effektivität, selbst bei geringer Wirkstoffdosierung.

Die dabei erzielte hohe Wirksamkeit ermöglicht einen sehr geringen Wirkstoffeinsatz, der zur Reduzierung des Eintrags von Waschmittelinhaltsstoffen in die aquatische Umwelt führt. Dadurch lassen sich Reinigungsprozesse im

Vergleich zu bekannten Methoden umweltfreundlicher und wirtschaftlicher gestalten.

Darüber hinaus ist es erfindungsgemäß möglich, hautfreundliche Reinigungsmittel herzustellen, was insbesondere bei der zunehmenden Häufigkeit von Hauterkrankungen bzw. von Allergien, hervorgerufen durch aggressive Reinigungsmittel, von Bedeutung ist.

Je nach gewünschter Anwendung können die erfindungsgemäßen grenzflächenaktiven Zusammensetzungen mit weiteren Inhaltsstoffen formuliert werden, um Reinigungsmittel auch für spezielle Anwendungen, wie etwa Abrasivreiniger, z.B. Scheuermilch, alkalische Formulierungen, z.B. Backofenreiniger, oder saure Formulierungen, z.B. Bad- oder WC-Reiniger, herzustellen.

Ein weiterer Vorteil bei der Verwendung der erfindungsgemäßen Zusammensetzung in Reinigungsmitteln besteht darin, dass aufgrund der hochmolekularen Struktur nach wiederholten Reinigungsprozessen eine Versiegelung der gereinigten Flächen beobachtet wird und somit eine geringere Wiederanschmutzung erfolgt. Dies macht auch eine leichtere Nachfolgereinigung möglich.

Die hohe Substantivität der erfindungsgemäßen Formulierung führt bei den gereinigten Flächen außerdem zu einem starken Glanzeffekt, der sich vor allem nach einem Trockenpolieren der gereinigten Flächen zeigt.

Die vorteilhaften Reinigungseigenschaften lassen sich gleichermaßen im Bereich der Waschmittel nutzen, sodass die erfindungsgemäße Zusammensetzung auch zur Herstellung von Waschmitteln verwendet werden kann.

Ein weiterer Gegenstand der Erfindung ist die Verwendung einer Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen, und
d) mindestens einer Aminosäure,

als Weichspüler, Schlichtungsmittel oder/und Egalisierungsmittel.

Die Kombination enthält mindestens einen Komplexbildner.

Neben der starken Reinigungswirksamkeit kann im Bereich der Textilwäsche vor allem die hohe Substantivität der erfindungsgemäßen grenzflächenaktiven Zusammensetzung ausgenutzt werden. Dies führt bei einer Behandlung von Textilien nach dem Waschvorgang zu einem weichmachenden Effekt unter Erhalt der hydrophilen, d.h. wasseraufnehmenden Eigenschaften. Durch den von der erfindungsgemäßen Zusammensetzung gezeigten Saugspüler-Effekt erfolgt eine Ausstattung von Textilien mit hydrophilem Charakter.

Handelsübliche Weichspüler rüsten Wäschestücke weich, griffig und antistatisch aus. Sie verändern jedoch die Textilien bezüglich deren natürlicher Eigenschaften der Wasseraufnahme. Da die Weichmachereffekte bei herkömmlichen Produkten überwiegend auf kationischen Aminderivaten basieren, werden die behandelten Wäschestücke wasserabweisend. Zudem sind die üblicherweise verwendeten kationischen Tenside oftmals toxikologisch bedenklich.

Erfindungsgemäß lassen sich hautfreundliche Weichspüler formulieren, die die hydrophilen Eigenschaften der Textilien verstärken, während die gewünschten Effekte, wie Griffigkeit, Weichheit, Antistatik usw., erhalten bleiben. Eine erfindungsgemäße Appretur verleiht den Wäschestücken außerdem verbesserte Bügeleigenschaften.

Die oben beschriebene grenzflächenaktive Zusammensetzung ist weiterhin zur Bildung von Mikrokapseln fähig. Dadurch können in Reinigungs- oder Waschmittelprodukten enthaltene Parfümöle fixiert und gleichmäßig über einen längeren Zeitraum freigegeben werden. Den Wäschestücken wird somit ein länger anhaltender Frischeduft verliehen.

Da die oben beschriebene grenzflächenaktive Zusammensetzung neben einer sehr guten Reinigungswirkung auch eine ausgezeichnete Netz-, Emulgier- und Dispergierwirkung aufweist, kann sie vorteilhaft in zahlreichen weiteren Anwendungen eingesetzt werden. Beispielsweise kann die Zusammensetzung als Basiswirkstoff in Netzmitteln (Wetting Agent) für die Textilfaserverarbeitung eingesetzt werden. Solche Netz- und Waschmittel können beispielsweise bei der Baumwollveredelung für folgende Prozesse eingesetzt werden:

Vorwaschen/Entschlichten; alkalisches Abkochen; Nachseifen von Färbungen und Drucken sowie Waschen/Fleckentfernung von produktionsbedingten Verunreinigungen.

Ein wesentlicher Vorteil ist, dass die erfindungsgemäße Zusammensetzung sehr schaumarm ist, in geringer Dosierung eingesetzt und leicht ausgespült werden kann. Dies führt zu kurzen Spülzeiten und einem geringen Wasserverbrauch. Weiterhin besitzen die erfindungsgemäß behandelten Textilfasern aufgrund der guten Hautverträglichkeit der oben beschriebenen grenzflächenaktiven Zusammensetzung ein deutlich geringeres Allergiepotenzial.

In einer weiteren bevorzugten Ausführungsform betrifft die Erfindung die Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen, und
d) mindestens einer Aminosäure,

zur Herstellung von kosmetischen Produkten.

Die oben beschriebenen grenzflächenaktiven Zusammensetzungen eignen sich auch für den Einsatz als waschaktive Substanzen in kosmetischen Produkten, die insbesondere der Reinigung dienen. Kosmetische Produkte auf Basis der erfindungsgemäßen grenzflächenaktiven Zusammensetzung sind nicht tränenreizend, rückfettend, nicht austrocknend, sehr mild und gut verträglich. Kosmetische Produkte, die unter Verwendung der oben beschriebenen grenzflächenaktiven Zusammensetzung hergestellt werden können, umfassen beispielsweise Duschgele, Handwaschgele, Zahnpasta, Badezusätze, Reinigungslotionen, Cremes etc. Da die erfindungsgemäße grenzflächenaktive Zusammensetzung als waschaktive Substanz nicht tränenreizend ist, kann sie auch vorteilhaft in Kinder- bzw. Babyprodukten eingesetzt werden. Zudem sind die auf Basis der erfindungsgemäßen grenzflächenaktiven Zusammensetzung hergestellten kosmetischen Produkte rückfettend und verhindern das Austrocknen der Haut. Diese Eigenschaften führen auch zu der in determatologischen Untersuchungen nachgewiesenen sehr guten Hautverträglichkeit.

Produkte für die Hautpflege, wie etwa Körperlotionen oder Cremes, können auf einfache Weise unter Verwendung der erfindungsgemäßen grenzflächenaktiven Zusammensetzung als Emulgiermittel unter Zusatz pflanzlicher Öle, z.B. Weizenkeimöl, hergestellt werden.

Ein weiterer Gegenstand der vorliegenden Erfindung ist die Verwendung einer oberflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff oder/und einem anionischen oder nicht-ionischen Tensid, und
c) mindestens einem Komplexbildner oder/und einer Verbindung, die in der Lage ist, mehrwertige Ionen auszufällen,

im Pflanzenschutz als Netzmittel.

Das ampholytische Protein weist keine proteolytische Enzymaktivität auf. Die Kombination enthält mindestens einen Komplexbildner.

Auf Basis der erfindungsgemäßen grenzflächenaktiven Zusammensetzung lassen sich umweltverträgliche Netzmittel herstellen. Solche Netzmittel sorgen beispielsweise für eine gleichmäßigere Verteilung von aufzubringenden Pflanzenbehandlungsmitteln, wie z.B. Pflanzenschutz- oder Düngemitteln. Durch die gleichmäßigere Benetzung der Wirkstoffformulierung kann bei der Bekämpfung von Schädlingen, wie etwa bei der Bekämpfung des Birnenblattsaugers, eine erkennbare Erhöhung der Abtötungsquote erzielt werden. Die Netzmittel auf Basis der erfindungsgemäßen grenzflächenaktiven Zusammensetzung sind für Pflanzenkulturen sehr gut verträglich, da sie auf organischen Inhaltsstoffen basieren und können aufgrund ihrer Zusammensetzung sogar als Nährstofflieferant, insbesondere als Stickstofflieferant für die behandelten Pflanzen fungieren.

Neben der Effektivitätssteigerung von Pflanzenschutzmitteln können die erfindungsgemäßen Netzmittel auch einen Abwascheffekt aufweisen, z.B. hinsichtlich Honigtau, den klebrigen, honiggelben Ausscheidungen der Birnenblattsauger.

Die erfindungsgemäßen Zusammensetzungen sind weiterhin als Netzmittel in Bewässerungssystemen für Kulturpflanzen einsetzbar, beispielsweise bei der Überkronenberegnung oder der Tröpfchenbewässerung. Die grenzflächenaktiven Eigenschaften der Zusammensetzung verhindern dabei die Tröpfchenbildung auf den Oberflächen der Pflanzen, z.B. Blätter, und vermeiden dadurch die Entstehung von Blattspritzflecken durch eine nach der Beregnung auftretende Sonneneinstrahlung. Dies hat einen besonders im Zierpflanzenbau erwünschten verbesserten Blattglanz zur Folge. Bei einer Bewässerung nach dem Ebbe-Flut-System wird durch einen Kapillareffekt ein gleichmäßiger Wasseranstieg in der Blumenerde bewirkt.

Darüber hinaus wird erfindungsgemäß nach der Bewässerung aufgrund der starken Reduzierung der Oberflächenspannung des Wassers eine sehr gleichmäßige Verteilung der Feuchtigkeit in der Erde erreicht.

Sehr vorteilhaft gegenüber konventionellen Netzmitteln auf synthetischer Basis für den Einsatz der erfindungsgemäßen Zusammensetzung in den oben beschriebenen Anwendungen ist die gute Pflanzenverträglichkeit sowie die vollkommene biologische Abbaubarkeit sowie das nichtschäumende Verhalten. Darüber hinaus stellen Rückstände der beschriebenen Formulierungen auf Nutzpflanzen aufgrund der Lebensmittelechtheit kein toxikologisches Problem dar.

Die Erfindung betrifft schließlich noch die Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff oder/und einem anionischen oder nicht-ionischen Tensid, und
c) mindestens einem Komplexbildner oder/und einer Verbindung, die in der Lage ist, mehrwertige Ionen auszufällen,

zur Bodensanierung, als Additiv im Baubereich oder/und als Additiv für Anstrichfarben oder Holzschutzmittel.

Das ampholytische Protein weist keine proteolytische Enzymaktivität auf. Die Kombination enthält mindestens einen Komplexbildner.

Erfindungsgemäß ist es möglich, große Mengen Öl in Wasser zu lösen. Viele organische Schadstoffe, wie etwa Benzin, Altöl, Schmierfette, halogenierte, aromatische Verbindungen und polycyclische Kohlenwasserstoffe (PAK), lassen sich nur schlecht in Wasser lösen und reichern sich deshalb an festen Grenzflächen an. Allein im Gebiet der Bundesrepublik Deutschland sind bisher etwa 80.000 Altlastenflächen erfasst, auf denen früher Tankstellen, fettverarbeitende Industriebetriebe oder Raffinerien betrieben wurden. Viele dieser Grundstücke sind mit hydrophoben, unpolaren Substanzen verseucht. Erfindungsgemäß lassen sich die Schadstoffe, insbesondere Fette oder Öle, aus kontaminierten Erdböden auf umweltfreundliche Art auswaschen, sodass eine erneute Nutzung dieser Flächen erfolgen kann. Die erfindungsgemäße Zusammensetzung kann somit die Grundlage für schonende Sanierungskonzepte bilden.

Weiterhin ist auch eine Anwendung als Additiv im Baubereich möglich. Erfindungsgemäß lassen sich umweltfreundliche und baubiologisch hochwertige Grundierkonzentrate für die Grundierung von Putzen sowohl im Innen- als auch im Außenbereich herstellen. Die erfindungsgemäße grenzflächenaktive Zusammensetzung wird im Allgemeinen Grundiermitteln in geringen Anteilen von etwa 0,1 Gew.-% bis 2 Gew.-% als Additiv zugesetzt und verleiht den Produkten ein besseres Eindringvermögen, insbesondere bei porösen, saugfähigen Flächen. In Verbindung mit Silikaten kann zudem eine deutliche Verfestigung von oberflächig mürben Untergrundbereichen erhalten werden.

Aufgrund der Verwendung natürlicher und für den Menschen zum Verzehr geeigneter Rohstoffe ist das Additiv darüber hinaus zur Herstellung lebensmittelechter Grundiermittel geeignet.

Die erfindungsgemäße Zusammensetzung kann aufgrund ihrer Eigenschaften auch als Dispergier- bzw. Emulgiermittel bei der Herstellung von Lebensmitteln eingesetzt werden. Außerdem können auf Basis der beschriebenen Zusammensetzung aus ampholytischem Protein und polaren polymeren Naturstoffen unter Verwendung bestimmter Komplexbildner und Feuchthaltemittel lebensmittelechte wasserlösliche Folien hergestellt werden.

Die beschriebene grenzflächenaktive Zusammensetzung kann weiterhin als Additiv bei der Herstellung von umweltverträglichen Anstrichfarben, beispielsweise auf Kalk- oder Kreidebasis, verwendet werden, wobei insbesondere die Wischfestigkeit gegenüber herkömmlichen Kalkfarben verbessert wird. Darüber hinaus führt das erfindungsgemäße Additiv zu einer deutlichen Verbesserung der Streichbarkeit.

Die Erfindung wird durch die folgenden Beispiele weiter erläutert.

**Beispiel 1**

**Formulierungen für verschiedene Anwendungsgebiete**

**Vergleichsbeispiel 1.1**

**Formulierung für einen Universalreiniger**

A

| | |
|---|---|
| 1,20 % | Tri-Na-citrat, dihydrat |
| 1,00 % | der grenzflächenaktiven Zusammensetzung aus z.B. 50 Gew-% Magermilchpulver, 25 Gew.-% CMC und 25 Gew.-% Amylopectin |
| 0,20 % | Parfümöle, z.B. Citroterpene |
| 0,10 % | Konservierung, z.B. Methylparaben |
| 0,05 % | Farbstoff |
| 97,45 % | entionisiertes Wasser |

B

| | |
|---|---|
| 3,20 % | der grenzflächenaktiven Zusammensetzung aus z.B. 35 Gew.-% Tri-Natriumcitrat, 35 Gew.-% Di-Natriumoxalat, 16 Gew.-% hydrolysiertes Milcheiweiß, 7 Gew.-% CMC und 7 Gew.-% Amylopectin |
| 0,20 % | Parfümöle, wie z.B. Citroterpen |
| 0,10 % | Konservierung, z.B. Methylparaben |
| 0,05 % | Farbstoff |
| 96,45 % | entionisiertes Wasser |

Ein solcher Reiniger wird vorteilhafterweise in Dosierungen von 10 bis 20 ml auf 2 bis 4 l Wasser eingesetzt, kann bei speziellen Verschmutzungen aber auch unverdünnt oder in höheren Konzentrationen verwendet werden.

**Vergleichsbeispiel 1.2**

**Formulierung für ein Waschmittel**

| | |
|---|---|
| 35 % | der grenzflächenaktiven Zusammensetzung aus z.B. 85 Gew.-% Di-Natriumoxalat, 8 Gew.-% Magermilchpulver, 3,5 % Gew.-% CMC und 3,5 Gew.-% Guarkernmehl |
| 35 % | Natrium-metasilikat, 5-hydrat |
| 30 % | Natriumpercarbonat |

Bei Buntwäsche konnte unter Anwendung dieser Formulierung eine gute Waschkraft bei sehr guter Farbbrillanz erreicht werden. Die Formulierung für ein Bunt- bzw. Vollwaschmittel kann beispielsweise mit einer Dosierung von ca. 60 g pro Waschmaschinenfüllung angewendet werden. Eine Parfümierung kann nach Belieben vorgenommen werden.

**Vergleichsbeispiel 1.3**

**Formulierung für ein Weichspülerkonzentrat**

A

| | |
|---|---|
| 2,40 % | Glycerinmonostearat |
| 0,60 % | Natriumlaurylethersulfat (28 %) |
| 0,40 % | der grenzflächenaktiven Zusammensetzung aus z.B. 50 Gew-% Ammoniumcaseinat, 25 Gew.-% Xanthan und 25 Gew.-% Guarkernmehl |
| 0,50 % | Parfüm |
| 0,10 % | Konservierung, z.B. Methylparaben oder Benzylalkohol |
| 96,00 % | entionisiertes Wasser |

B

| | |
|---|---|
| 2,40 % | Glycerinmonostearat |
| 0,60 % | der grenzflächenaktiven Zusammensetzung aus z.B. 35 Gew.-% Tri-Na-citrat, 35 Gew.-% Caseinhydrolysat, 15 Gew.-% Xanthan und 15 Gew.-% Guarkernmehl |
| 0,50 % | Natriumlaurylethersulfat (28 % Wirkstoffgehalt) |
| 0,40 % | Parfüm |
| 0,10 % | Konservierung, z.B. Methylparaben oder Benzylalkohol |
| 96,00 % | entionisiertes Wasser |

Die beschriebene Formulierung verleiht den behandelten Wäschestücken eine gute Griffigkeit, Weichheit und

Antistatik unter Erhalt der Saugfähigkeit.

**Vergleichsbeispiel 1.4**

**Formulierung für die Textilfaserverarbeitung**

| | |
|---|---|
| 1,20 % | der grenzflächenaktiven Zusammensetzung aus z.B. 35 Gew.-% Natriumcaseinat, 30 Gew.-% Xanthan, 20 Gew.-% Di-Natriumoxalat und 15 Gew.% Tri-Natriumcitrat |
| 0,80 % | Natriumhydrogencarbonat |
| 0,20 % | Konservierung, z.B. Phenoxyethanol |
| 97,80 % | entionisiertes Wasser |

Eine solche Formulierung kann beispielsweise zur Baumwollveredelung eingesetzt werden. Beim Einsatz im Prozess des Vorwaschens und Entschlichtens der Baumwollfaser wurde verglichen mit einem herkömmlichen, synthetischen Produkt ein entsprechender Weißgrad und eine sehr gute Saugfähigkeit sowie ein sehr guter Griff erreicht. Beim Nachseifen von Färbungen, z.B. Reaktivfärbungen, führt der Einsatz der aufgeführten Formulierung zu einer sehr guten Waschechtheit der gefärbten Fasern.

**Vergleichsbeispiel 1.5**

**Formulierung als kosmetisches Reinigungsprodukt**

A

| | |
|---|---|
| 1,44 % | der grenzflächenaktiven Zusammensetzung aus z.B. 50 Gew-% Magermilchpulver und 50 Gew.-% Carboxymethylcellulose |
| 1,44 % | Sorbit |
| 0,36 % | Tri-Natriumcitrat |
| 0,17 % | Methylparaben (Konservierungsmittel) |
| 0,09 % | Propylparaben (Konservierungsmittel) |
| 96,50 % | Wasser |

Selbst bei Allergikern sowie bei MCS-Erkrankten (Multiple Chemical Sensitivity) zeigte die Anwendung der beschriebenen Beispielrezeptur auf Basis der erfindungsgemäßen waschaktiven Substanzkombination eine sehr gute Verträglichkeit. Produkte für die Hautpflege, wie etwa Körperlotionen, können auf einfache Weise durch Einemulgieren wertvoller pflanzlicher Öle, z.B. Weizenkeimöl, in die aufgeführte Rezeptur hergestellt werden.

**Beispiel 1.6**

**Formulierung als Netzmittel für den Pflanzenschutz im Obstbau**

| | |
|---|---|
| 6,00 % | der erfindungsgemäßen grenzflächenaktiven Zusammensetzung aus z.B. 83 Gew.-% Tri-Natriumcitrat, 8,5 Gew.-% Magermilchpulver, 8,5 Gew.-% Xanthan |
| 0,30 % | Konservierung, z.B. Isothiazolinone |
| 93,70 % | entionisiertes Wasser |

**Beispiel 1.7**

**Formulierung als Additiv im Baubereich**

| | |
|---|---|
| 2,40 % | Natrium-metasilikat, 5-hydrat |
| 1 ,00 % | der erfindungsgemäßengrenzflächenaktiven Zusammensetzung, aus z.B. 50 Gew-% Natriumcaseinat und 50 Gew.-% Carboxymethylcellulose |
| 96,60 % | Wasser |

Diese Formulierung, in der der Zusatz von weiteren Additiven nicht erforderlich ist, stellt ein wasserverdünnbares, lösungsmittelfreies und transparentes Grundiermittel dar, das ein sehr gutes Eindringvermögen, vor allen Dingen bei der Anwendung auf Betonflächen sowie bei saugenden, mineralischen Untergründen, wie etwa Kalk-Zement-Leichtputz, besitzt.

Die Formulierung kann u.a. erfolgreich zur Verfestigung von pudrigen Altrestbeschichtungen, z.B. abgewaschenen Leimfarbenflächen, eingesetzt werden. Sie egalisiert die Saugfähigkeit bei unterschiedlich saugenden Untergründen und verhindert Glanzstellen beim nachfolgenden Aufbringen von Farbanstrichen. Die Verarbeitung der beschriebenen Formulierung in mineralischen Putzen führt zu einem schnelleren Anziehen von neu aufgetragenen Putzen, was eine schnellere Nachbearbeitung ermöglicht. Darüber hinaus besitzt das Grundiermittel eine hohe Gas- bzw. Wasserdampfdurchlässigkeit.

**Beispiel 1.8**

**Formulierung für eine Innenwandfarbe Grundweiß**

| | |
|---|---|
| 44,00 % | Kreide |
| 4,00 % | Pflanzenöl, z.B. Sonnenblumenöl |
| 1,25 % | der erfindungsgemäßen grenzflächenaktiven Zusammensetzung aus z.B. 60 Gew.-% Natrium-metasilikat, 5-hydrat, 20 Gew.-% Säurecasein und 20 Gew.-% Xanthan |
| 0,15 % | Konservierung, z.B. Methylparaben |
| 50,60 % | entionisiertes Wasser |

Diese Formulierung entspricht baubiologischen Anforderungen. Die Formulierung verharzt im Gegensatz zu üblichen Kreide- oder Kalkfarben in einem Zeitraum von ca. 3 Wochen ab dem Zeitpunkt des Auftrags und ist äußerst wischfest.

**Beispiel 1.9**

**Formulierung für einen sauren Reiniger**

| | |
|---|---|
| 6,00 % | Citronensäure |
| 1,00% | der erfindungsgemäßen grenzflächenaktiven Zusammensetzung aus z.B. 28 Gew.-% Magermilchpulver, 28 Gew. % Xanthan, 28 Gew.-% Guarkernmehl und 16 Gew.-% di-Natriumoxalat |
| 1,00 % | Mononhatriumglutamat |
| 0,40 % | Parfümöl |
| 0,20 % | Konservierung, z.B. Benzoesäure |
| 0,05 % | Farbstoff |
| 91,35 % | entionsiertes Wasser |

**Beispiel 1.10**

**Formulierung für einen Abrasivreiniger (Scheuermilch)**

| | |
|---|---|
| 34,00 % | amorphe Kieselsäure |
| 5,10 % | der erfindungsgemäßen grenzflächenaktiven Zusammensetzung aus z.B. 58 Gew.-% Molkenproteinhydrolysat, 7 Gew.-% Xanthan und 35 Gew.% Citronensäure |
| 1,50 % | Mononatriumglutamat |

0,20 % Konservierung, z.B. Methylparaben
59,20 % entionsiertes Wasser

**Beispiel 2**

**Biologische Abbaubarkeit**

Es wurde die biologische Abbaubarkeit der erfindungsgemäßen Zusammensetzungen umfassend ein ampholytisches Protein sowie einen polaren anionischen oder nicht-ionischen polymeren Naturstoff im $CO_2$-Entwicklungstest nach 67/548/EWG C.4-C untersucht. Die Prüfkonzentration betrug 96,8 mg/l (entsprechend 30 mg TOC/l). Zur Probenvorbereitung wurde die Zusammensetzung als Pulver direkt in die Testgefäße gegeben. Die Versuchstemperatur betrug 22 $\pm$ 1 °C. Bei diesen Untersuchungen wurde festgestellt, dass die erfindungsgemäße grenzflächenaktive Zusammensetzung leicht biologisch abbaubar ist, mit 92 % $ThCO_2$ in 10 Tagen und 100 % in 21 Tagen.

**Beispiel 3**

**Reinigung und Pflege von Kraftfahrzeugen**

Die erfindungsgemäße grenzflächenaktive Zusammensetzung wurde als Lackreiniger für Kraftfahrzeuge verwendet. Dabei zeigten sich hervorragende Reinigungsergebnisse. Auch hartnäckige Verschmutzungen konnten entfernt werden.

Die Zusammensetzung kann auch als Produkt für die Autowäsche formuliert werden und führt zu hervorragenden Reinigungsergebnissen. Gewöhnungsbedürftig ist lediglich, dass praktisch kein Schaum entsteht.

Die Zusammensetzung kann auch als Scheibenreiniger eingesetzt werden und führt zu außerordentlich guten Ergebnissen. Dies ist insbesondere bemerkenswert, da der Zusatz von hautreizenden Alkoholen oder sonstigen organischen Lösungsmitteln nicht erforderlich ist.

**Beispiel 4**

**Hautverträglichkeit kosmetischer Produkte**

**Beispiel 4.1**

**Patch-Test am Menschen**

Mit dem Patch-Test am Menschen wurde das irritative, d.h. hautreizende Potenzial untersucht. Unter den Bedingungen dieses Test zeigte kein einziger von 50 Probanden eine positive Hautreaktion, während das als Positivkontrolle verwendete konventionelle Tensid Natriumlaurylsulfat (SDS) unter den gleichen Testbedingungen bei 13 Testpersonen eine positive, d.h. irritative Hautreaktion zeigte.

**Beispiel 4.2**

**Modifizierter Duhring-Kammer-Test**

Auch die Prüfung auf Hautreizung beim Menschen im modifizierten During-Kammer-Test, einem Testmodell, mit dem die Irritationspotenziale verschiedener dermatologischer und kosmetischer Produkte erfasst werden, bestätigte die gute Hautverträglichkeit der erfindungsgemäßen grenzflächenaktiven Zusammensetzung.

Beispiel 5

**Testung auf membranirritierende Eigenschaften**

**Experimentalteil**

**HET-CAM-Test (Testung auf membranirritierende Eigenschaften)**

Herstellung der Testlösungen:

Eine erfindungsgemäße Zusammensetzung, im Beispiel als Tensoprot M bezeichnet, wurde in 0,9 %-iger steriler Kochsalzlösung gelöst (2 % m/V).
Als Positivkontrolle wurde Natriumdodecylsulfat in 0.9 %-iger steriler Kochsalzlösung gelöst (0,5 % m/V).
Als Blindkontrolle wurde 0,9 %-ige sterile Kochsalzlösung verwendet.

Durchführung des HET-CAM-Assays:

Befruchtete Hühnereier wurden in horizontaler Lage unter mehrmaligem Drehen 65 bis 70 h bei 37 °C und 80 % relativer Luftfeuchtigkeit inkubiert.
Die Eier wurden von der stumpfen Seite her geöffnet, wobei zunächst an der spitzen Seite des Eies zur Absenkung des Eidotters 15 bis 20 ml Eiweiß aus dem Ei abgesaugt wurden. Die Eierschale wurde dann auf ungefähr 2/3 der Höhe angeritzt und mit der Pinzette entfernt. Nach Entfernen der Luftsackmembran wurden die präparierten Eier mit Frischhaltefolie bedeckt und zur weiteren Entwicklung ca. 75 h bei 37 °C und 80 % rel. Luftfeuchtigkeit inkubiert.
Am zehnten Inkubationstag wurden zur Testung 0,2 ml Lösung auf die Chorionallantoismembran (CAM) gegeben. Für jede Lösung wurden 12 bis 14 Eier verwendet. Nach Applikation der Lösungen wurden sowohl die CAM als auch die Blutgefäße einschließlich des Kapillarnetzes und die Eiweißkoagulation über einen Zeitraum von 5 Minuten beobachtet. Längere Beobachtungszeiten führten zu keinen zusätzlichen Informationen. Registriert wurden Kapillarveränderungen (Hyperämie), Hämorrhagie und Koagulation. Jeder Test wurde zweimal durchgeführt.

Auswertung:

Die Reizreaktion wurde nach 5 Minuten wie folgt bewertet:

| | |
|---|---|
| Nicht reizend: | Keine Reizung. Aussehen der CAM wie nach 5-minütiger Behandlung mit 0,9 %iger Kochsalzlösung. |
| Leicht reizend: | Kapillargefäßveränderung und leichte Hämorrhagie wie nach 1-minütier SDS-Behandlung. |
| Stark reizend: | Starke Hämorrhagie der Blutgefäße wie nach 5-minütiger SDS-Behandlung |
| Sehr stark reizend: | Eiweißkoagulation. |

**Ergebnisse der Untersuchungen**

| Lösung | Reizung | % |
|---|---|---|
| Blindlösung (0,9 % NaCl) | nicht reizend | 100 (+/- 0) |
| Testlösung (Tensoprot M, 2 %) | nicht reizend | 100 (+/- 0) |
| Positivkontrolle (0,5 % SDS) | stark reizend | 100 (+/- 0) |

**Zusammenfassung**

Tensoprot M zeigt keine mebranirritierenden Eigenschaften.

## Patentansprüche

1. Grenzflächenaktive Zusammensetzung, umfassend eine Kombination aus

   a) mindestens einem ampholytischen Protein oder Peptid, welches hydrophile Bereiche enthält oder einem Proteinhydrolysat, welche keine proteolytische Enzymaktivität besitzen,
   b) mindestens einem polaren anionischen oder/und nicht-ionischen polymeren Naturstoff.
   c) mindestens einem Komplexbildner oder/und einer Verbindung, die in der Lage ist, mehrwertige lonen auszufällen und
   d) mindestens einerAminosäure.

2. Zusammensetzung nach Anspruch 1,
**dadurch gekennzeichnet,**
**dass** das ampholytische Protein sowohl hydrophile als auch hydrophobe Bereiche enthält.

3. Zusammensetzung nach einem der vorhergehenden Ansprüche,
**dadurch gekennzeichnet,**
**dass** das ampholytische Protein mindestens 10 basisch wirkende Gruppierungen und mindestens 10 sauer wirkende Gruppierungen enthält.

4. Zusammensetzung nach einem der vorhergehenden Ansprüche,
**dadurch gekennzeichnet,**
**dass** sie als ampholytisches Protein ein aus einem ampholytischen Protein durch Hydrolyse hergestelltes Peptidgemisch enthält.

5. Zusammensetzung nach einem der vorhergehenden Ansprüche,
**dadurch gekennzeichnet,**
**dass** sie weiterhin ein Tensid oder/und Salz umfasst.

6. Zusammensetzung nach einem der vorhergehenden Ansprüche,
**dadurch gekennzeichnet,**
**dass** sie mindestens einen polaren anionischen polymeren Naturstoff, welcher negative Ladungen enthält und/oder ein anionisches Tensid umfasst, wodurch der isoelektrische Punkt der Gesamtzusammensetzung bezogen auf den isoelektrischen Punkt des enthaltenen ampholytischen Proteins verschoben wird.

7. Zusammensetzung nach einem der vorhergehenden Ansprüche,
**dadurch gekennzeichnet,**
**dass** das ampholytische Protein ausgewählt wird aus Milchproteinen, Weizenproteinen, Sojaproteinen, Lupinenproteinen oder/und Erbsenproteinen oder aus daraus durch Hydrolyse gebildeten Peptidgemischen.

8. Zusammensetzung nach Anspruch 7,
**dadurch gekennzeichnet,**
**dass** sie als ampholytisches Protein aufgeschlossenes Milcheiweiß oder/und Magermilchpulver enthält oder daraus durch Hydrolyse gebildete Peptidgemische enthält.

9. Zusammensetzung nach einem der vorhergehenden Ansprüche,
**dadurch gekennzeichnet,**
**dass** sie einen polaren anionischen polymeren Naturstoff, insbesondere ein Polysaccharid, z.B. Xanthan, Carboxymethylcellulose oder/und Phosphatstärke enthält.

10. Zusammensetzung nach einem der vorhergehenden Ansprüche,
**dadurch gekennzeichnet,**
**dass** sie einen polaren nicht-ionischen polymeren Naturstoff, insbesondere Guarkernmehl oder/und Amylopectin, enthält.

11. Konzentrat, enthaltend eine grenzflächenaktive Zusammensetzung nach einem der Ansprüche 1 bis 10 in einer Konzentration von $\geq$ 1 Gew.-%.

**12.** Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff
c) mindestens einemkomplexbildner oder/und einer Verbindung, die in der Lage ist, mehrwertige Ionen auszufällen und
d) mindestens einerAminosäure

als Reinigungsmittel, Waschmittel, Emulgiermittel oder/und Dispergiermittel.

**13.** Verwendung nach Anspruch 12,
**dadurch gekennzeichnet,**
**dass** die Zusammensetzung weiterhin eine Fettsäureverbindung umfasst.

**14.** Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen und
d) mindestens einer Aminosäure

als Weichspüler, Schlichtungsmittel oder/und Egalisierungsmittel.

**15.** Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen und
d) mindestens einer Aminosäure

zur Herstellung von kosmetischen Produkten.

**16.** Verwendung nach Anspruch 15 als Haarshampoo, Schaumbad, Ölbad, Creme oder/und Lotion.

**17.** Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff oder/und einem anionischen oder nicht-ionischen Tensid, und
c) mindestens einem Komplexbildner oder/und einer Verbindung, die in der Lage ist, mehrwertige Ionen auszufällen,

im Pflanzenschutz als Netzmittel.

**18.** Verwendung einer grenzflächenaktiven Zusammensetzung, umfassend eine Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder nicht-ionischen polymeren Naturstoff oder/und einem anionischen oder nicht-ionischen Tensid, und
c) mindestens einem Komplexbildner oder/und einer Verbindung, die in der Lage ist, mehrwertige Ionen auszufällen,

zur Bodensanierung, als Additiv im Baubereich oder/und als Additiv für Anstrichfarben oder Holzschutzmittel.

**19.** Verwendung einer Kombination aus

a) mindestens einem ampholytischen Protein oder Peptid, welches hydrophile Bereiche enthält oder einem Proteinhydrolysat, welche keine proteolytische Enzymaktivität besitzen,
b) mindestens einem polaren anionischen oder/und nicht-ionischen polymeren Naturstoff,
c) mindestens einem Komplexbildner oder/und einer Verbindung, die in der Lage ist, mehrwertige Ionen auszufällen, und
d) mindestens einer Aminosäure

als grenzflächenaktive Zusammensetzung.

## Claims

**1.** Surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance,
c) at least one complex-forming agent and/or a compound that is able to precipitate polyvalent ions, and
d) at least one amino acid.

**2.** Composition according to claim 1, **characterised in that** the ampholytic protein contains both hydrophilic and hydrophobic regions.

**3.** Composition according to one of the preceding claims, **characterised in that** the ampholytic protein contains at least 10 alkaline acting groups and at least 10 acidic acting groups.

**4.** Composition according to one of the preceding claims, **characterised in that** it contains as ampholytic protein a peptide mixture produced from an ampholytic protein by hydrolysis.

**5.** Composition according to one of the preceding claims, **characterised in that** it furthermore contains a surfactant and/or a salt.

**6.** Composition according to one of the preceding claims, **characterised in that** it comprises at least one polar anionic polymeric natural substance that contains negative charges and/or comprises an anionic surfactant, whereby the isoelectric point of the overall composition is displaced with respect to the isoelectric point of the contained ampholytic protein.

**7.** Composition according to one of the preceding claims, **characterised in that** the ampholytic protein is selected from milk proteins, wheat proteins, soya proteins, lupin proteins and/or pea proteins or from peptide mixtures formed therefrom by hydrolysis.

**8.** Composition according to claim 7, **characterised in that** it contains, as ampholytic protein, a solubilised milk protein and/or skimmed milk powder, or contains peptide mixtures formed therefrom by hydrolysis.

**9.** Composition according to one of the preceding claims, **characterised in that** it contains a polar anionic polymeric natural substance, in particular a polysaccharide, e.g. xanthan, carboxymethylcellulose and/or phosphate starch.

**10.** Composition according to one of the preceding claims, **characterised in that** it contains a polar non-ionic polymeric natural substance, in particular guar flour and /or amylopectin.

**11.** Concentrate containing a surface-active composition according to one of claims 1 to 10 in a concentration of $\geq 1$ wt.%.

**12.** Use of a surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance,
c) at least one complex-forming agent and/or a compound that is able to precipitate polyvalent ions, and
d) at least one amino acid

as a cleaning agent, detergent, emulsifier and/or dispersant.

**13.** Use according to claim 12, **characterised in that** the composition also contains a fatty acid compound.

**14.** Use of a surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance,
c) at least one complex-forming agent and/or a compound that is able to precipitate polyvalent ions, and
d) at least one amino acid

as a softener, sizing material and/or levelling agent.

**15.** Use of a surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance,
c) at least one complex-forming agent and/or a compound that is able to precipitate polyvalent ions, and
d) at least one amino acid

for the production of cosmetic products.

**16.** Use according to claim 15, as a hair shampoo, foam bath, oil bath, crème and/or lotion.

**17.** Use of a surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance, and
c) at least one complex-forming agent and/or a compound that is able to precipitate polyvalent ions,

as a wetting agent in plant protection.

**18.** Use of a surface-active composition, comprising a combination of

a) at least one ampholytic protein or peptide that contains hydrophilic regions, or a protein hydrolysate that has no proteolytic enzyme activity,
b) at least one polar anionic and/or non-ionic polymeric natural substance, and
c) at least one complex-forming agent and/or a compound that is able to precipitate polyvalent ions

for soil rehabilitation, as an additive in the building/construction sector and/or as an additive for coating compounds, paints or wood preservatives.

**19.** Use of a combination of

a) at least one ampholytic protein or peptide that contains hydrophilic regions, or a protein hydrolysate that has no proteolytic enzyme activity,
b) at least one polar anionic and/or non-ionic polymeric natural substance,
c) at least one complex-forming agent and/or a compound that is able to precipitate polyvalent ions, and
d) at least one amino acid

as a surface-active composition.

**Revendications**

1. Composition tensioactive, comprenant une combinaison de

   a) au moins une protéine ou un peptide ampholyte contenant des domaines hydrophiles ou un hydrolysat de protéine, qui ne possèdent pas d'activité enzymatique protéolytique,
   b) au moins une substance naturelle polymère polaire anionique et/ou non ionique,
   c) au moins un agent complexant et/ou un composé qui est en mesure de précipiter des ions polyvalents, et
   d) au moins un aminoacide.

2. Composition selon la revendication 1, **caractérisée en ce que** la protéine ampholyte contient à la fois des domaines hydrophiles et hydrophobes.

3. Composition selon l'une des revendications précédentes, **caractérisée en ce que** la protéine ampholyte contient au moins 10 groupes à action basique et au moins 10 groupes à action acide.

4. Composition selon l'une des revendications précédentes, **caractérisée en ce qu'**elle contient comme protéine ampholyte un mélange de peptides préparé par hydrolyse d'une protéine ampholyte.

5. Composition selon l'une des revendications précédentes, **caractérisée en ce qu'**elle comprend en outre un agent tensioactif et/ou un sel.

6. Composition selon l'une des revendications précédentes, **caractérisée en ce qu'**elle comprend au moins une substance naturelle polymère anionique polaire qui contient des charges négatives et/ou un agent tensioactif anionique, grâce à quoi le point isoélectrique de l'ensemble de la composition est déplacé par rapport au point isoélectrique de la protéine ampholyte contenue.

7. Composition selon l'une des revendications précédentes, **caractérisée en ce que** la protéine ampholyte est choisie parmi les protéines de lait, les protéines de blé, les protéines de soja, les protéines de lupin et/ou les protéines de pois ou les mélanges de peptides formés par hydrolyse de ceux-ci.

8. Composition selon la revendication 7, **caractérisée en ce qu'**elle contient comme protéine ampholyte de l'albumine de lait dégradée et/ou de la poudre de lait écrémé ou des mélanges de peptides formés par hydrolyse de ceux-ci.

9. Composition selon l'une des revendications précédentes, **caractérisée en ce qu'**elle contient une substance naturelle polymère polaire anionique, en particulier un polysaccharide, par exemple du xanthane, de la carboxyméthylcellulose et/ou un phosphate d'amidon.

10. Composition selon l'une des revendications précédentes, **caractérisée en ce qu'**elle contient une substance naturelle polymère polaire non ionique, en particulier de la farine de graines de guar et/ou de l'amylopectine.

11. Concentré contenant une composition tensioactive selon l'une des revendications 1 à 10 à une concentration égale ou supérieure à 1 % en masse.

12. Utilisation d'une composition tensioactive comprenant une combinaison de

    a) au moins une protéine ou un peptide ampholyte contenant des domaines hydrophiles ou un hydrolysat de protéine, qui ne possèdent pas d'activité enzymatique protéolytique,
    b) au moins une substance naturelle polymère polaire anionique et/ou non ionique,
    c) au moins un agent complexant et/ou un composé qui est en mesure de précipiter des ions polyvalents, et
    d) au moins un aminoacide

    comme agent détergent, agent de lavage, agent émulsionnant et/ou agent dispersant.

13. Utilisation selon la revendication 12, **caractérisé en ce que** la composition comprend en outre un composé d'acide

gras.

**14.** Utilisation d'une composition tensioactive comprenant une combinaison de

a) au moins une protéine ou un peptide ampholyte contenant des domaines hydrophiles ou un hydrolysat de protéine, qui ne possèdent pas d'activité enzymatique protéolytique,
b) au moins une substance naturelle polymère polaire anionique et/ou non ionique,
c) au moins un agent complexant et/ou un composé qui est en mesure de précipiter des ions polyvalents, et
d) au moins un aminoacide

comme agent assouplissant, agent d'apprêtage et/ou agent d'égalisation.

**15.** Utilisation d'une composition tensioactive comprenant une combinaison de

a) au moins une protéine ou un peptide ampholyte contenant des domaines hydrophiles ou un hydrolysat de protéine, qui ne possèdent pas d'activité enzymatique protéolytique,
b) au moins une substance naturelle polymère polaire anionique et/ou non ionique,
c) au moins un agent complexant et/ou un composé qui est en mesure de précipiter des ions polyvalents, et
d) au moins un aminoacide

pour la préparation de produits cosmétiques.

**16.** Utilisation selon la revendication 15 sous forme de shampooing, bain moussant, huile de bain, crème et/ou lotion.

**17.** Utilisation d'une composition tensioactive comprenant une combinaison de

a) au moins une protéine ou un peptide ampholyte contenant des domaines hydrophiles ou un hydrolysat de protéine, qui ne possèdent pas d'activité enzymatique protéolytique,
b) au moins une substance naturelle polymère polaire anionique et/ou non ionique,
c) au moins un agent complexant et/ou un composé qui est en mesure de précipiter des ions polyvalents, et
d) au moins un aminoacide

comme agent mouillant dans le domaine phytosanitaire.

**18.** Utilisation d'une composition tensioactive comprenant une combinaison de

a) au moins une protéine ou un peptide ampholyte contenant des domaines hydrophiles ou un hydrolysat de protéine, qui ne possèdent pas d'activité enzymatique protéolytique,
b) au moins une substance naturelle polymère polaire anionique et/ou non ionique,
c) au moins un agent complexant et/ou un composé qui est en mesure de précipiter des ions polyvalents, et
d) au moins un aminoacide

pour l'assainissement des sols, comme additif dans le domaine de la construction et/ou comme additif pour des peintures ou des produits de protection du bois.

**19.** Utilisation d'une combinaison de

a) au moins une protéine ou un peptide ampholyte contenant des domaines hydrophiles ou un hydrolysat de protéine, qui ne possèdent pas d'activité enzymatique protéolytique,
b) au moins une substance naturelle polymère polaire anionique et/ou non ionique,
c) au moins un agent complexant et/ou un composé qui est en mesure de précipiter des ions polyvalents, et
d) au moins un aminoacide

comme composition tensioactive.